## Europäisches Patentamt

**European Patent Office**

**Office européen des brevets**

(11) Publication number: **0 296 118**
**A2**

# EUROPEAN PATENT APPLICATION

(21) Application number: **88810394.2**

(22) Date of filing: **10.06.88**

(51) Int. Cl.⁴: **A 23 D 5/00**

(30) Priority: **16.06.87 US 62927**

(43) Date of publication of application:
**21.12.88 Bulletin 88/51**

(84) Designated Contracting States:
**AT BE CH DE ES FR GB GR IT LI LU NL SE**

(71) Applicant: **WARNER-LAMBERT COMPANY**
**201 Tabor Road**
**Morris Plains New Jersey 07950 (US)**

(72) Inventor: **Geria, Navin Manohar**
**32 Mountainview Road**
**Warren New Jersey 07060 (US)**

(74) Representative: **Silbiger, Jakob, Dr.**
**c/o CAPSUGEL AG Münchensteinerstrasse 41**
**CH-4002 Basel (CH)**

(54) Oil compositions of increased viscosity and method of preparing same.

(57) Increased viscosity oil compositions have been prepared which comprise an oil and an inorganic complexing agent, said complexing agent being insoluble in oil and forming a viscosity increasing complex with the oil and a method for producing same.

EP 0 296 118 A2

Bundesdruckerei Berlin

Description

## OIL COMPOSITIONS OF INCREASED VISCOSITY AND METHOD OF PREPARING SAME

This invention relates to novel animal, vegetable and mineral oil compositions having increased viscosity and a method of making the same. These compositions comprise a major proportion of oil, an inorganic complexing agent capable of effectuating a viscosity increase without substantially effecting other properties of the oil.

Oil compositions of increased viscosity are used as a base component or starting material for the formation of a number of products.

Oil based products are useful in the food, pharmaceutical and cosmetic industries. In general, it is desirable to thicken or increase the viscosity of oils to facilitate the formation of suspensions, lotions, creams, greases, salves, ointments and butter or margarine like products.

Well known techniques utilized to increase oil viscosity include: emulsifying oils with an aqueous component; combining oil with a polymeric material or a higher melting wax having a long chain molecular structure; and hydrogenation of unsaturated oils to increase viscosity through molecular interaction. Each of these techniques has its own drawbacks. For example, emulsions contain an aqueous portion which may be detrimental to other desired ingredients such as sweeteners, flavors and medicaments. Addition of polymeric materials and waxes to oil generally requires heat which may degrade the oil or other components of the final product. Hydrogenation is expensive and produces saturated fats which are considered unacceptable for many ingestable products for health reasons.

It has been unexpectedly discovered that the addition of certain inorganic compounds to oil will produce a gel-like structure or complex within the oil. This gel-like structure or complex results in an increase in oil viscosity. The increase in viscosity is proportional to the concentration of inorganic compound.

In particular, it has been found that an oil composition having increased viscosity is produced from an admixture of an oil and an inorganic complexing agent. The inorganic complexing agent forms a complex with the oil, thereby increasing the viscosity of the oil without affecting the remaining oil properties.

In this specification, "oil" means any animal, vegetable or mineral oil, and includes such substances as marine oils. Marine oils include substances such as fish liver oils, fish oils, whale oil, seal oil and fish oils containing omega-3 fatty acids. All marine oils may contain omega-3 fatty acids.

Throughout the specification and claims the terms inorganic complexing agent and inorganic complexing compound shall be used interchangeably to mean an inorganic compound which forms a complex or is otherwise associated with the oil to produce a gel-like structure with oil.

While the invention is not to be limited to theoretical considerations, it is believed that the incorporation of the inorganic complexing agent into the oil provides a particle surface which interacts with the oil molecules in such a way that one portion of the oil molecule is attracted to and becomes associated or complexed with the particle surface while the remainder of the oil molecule extends away from the particle. This phenomenon produces what is termed a "solvated particle". The ends of the oil molecules that extend away from the solvated particle are available to interact with remaining oil or with other solvated particles of complexing agent. These interactions cause the oil and complexing agent mixture to develop a weak gel-like structure, thereby forming an oil composition having increased viscosity. The structure is believed analogous to that of a micelle in which the solid particle of inorganic complexing agent is held inside a pseudo-micelle.

The oils useful in the present invention are varied and may be of animal, vegetable or mineral origin. Methods of producing oils are known and not a subject of the present invention. Animal oils are derived from the organs and tissues of animals and may be collected through extraction, heating and/or expressing processes. Vegetable oils are usually derived from the seeds of various plants and are generally produced by extraction or pressing processes. Mineral oils are derived from petroleum and are recovered through various refining processes. Throughout the specification and claims, the term "oil" shall be defined as any oil of animal, vegetable or mineral origin in liquid form at the time of addition of the inorganic complexing agent.

The oils useful in the present invention may be food grade edible oils or nonedible oils. For example, food grade oils would be particularly useful in edible pharmaceuticals and food products. Nonedible oils would be useful in topical pharmaceuticals, cosmetics, personal care products and in lubricants.

Illustrative, nonlimiting examples of oils useful in the present invention include animal oils such as the marine oils: fish oil, whale oil, fish liver oil, seal oil, oils containing at least one omega-3 fatty acid and the like; vegetable oils such as castor oil, linseed oil, sunflower oil, soybean oil, olive oil, peanut oil, rapeseed oil, corn oil, safflower seed oil, cottonseed oil, coconut oil, palm oil, palm kernel oil, oils containing at least one omega-3 fatty acid and the like; mineral oils such as white mineral oil and the like. Any of the oils may be used individually or in mixtures.

The complexing agent is characterized by being insoluble in oil, being inorganic and by its ability to form a complex with the oil such that the viscosity of the oil is increased.

The inorganic complexing agents must be incorporated into the oil in particulate form. The particle size may vary widely but must be of adequate size to enable incorporation into the oil without exhibiting a gritty or sandy feel. In addition, the particle size must be adequate to enable complex formation with the oil. Exemplary average particle size ranges may be from about 0.1 micrometers to about 300 micrometers, preferably about 0.5 micrometers to about 150 micrometers and most preferably about 0.5 micrometers to about 100 micrometers.

The inorganic complexing agent may be selected from a wide range of compounds that are insoluble in oil and provide complexation of oil when admixed with oil. Exemplary inorganic complexing agents may be selected from the group consisting of magnesium trisilicate, magnesium hydroxide, calcium carbonate, calcium silicate, a co-dried gel of aluminum hydroxide and magnesium carbonate, magnesium carbonate, aluminum hydroxide, ground limestone, ground oyster shells, and mixtures thereof.

The change in oil viscosity with increasing complexing agent concentration is a continuum. Free flowing liquids are formed at low complexing agent concentrations and thick, essentially nonflowing compositions having a texture like that of petroleum jelly are formed at the higher complexing agent concentrations. The "gel-like" structure of the present invention has very weak elastic properties and will readily deform when a slight force is applied.

The ratio of inorganic complexing agent to oil will determine the viscosity or degree of thickening of the oil. A complexing agent to oil ratio of about 1:99 to about 1:10 will produce liquids having a gradual increase in viscosity. Complexing agent to oil ratios of about 1:9 to about 1:3 will produce liquids with the consistency of syrups. Complexing agent to oil ratios of about 1:2.5 to about 1:1 will produce semisolids having the consistency of creams to ointments. Complexing agent to oil ratios greater than about 1:1 will produce solids. The texture of the solids will become granular and change to a powder as the complexing agent to oil ratio increases beyond about 1.2:1. In general, complexing agent to oil ratios of about 1:99 to about 1.2:1 will produce compositions useful in the present invention.

The compositions of the present invention are prepared by admixing the inorganic complexing agent with the oil until a uniform homogeneous mixture is obtained. The inorganic complexing agent interacts with the oil to increase viscosity and form a gel-like structure.

The admixing of oil and inorganic complexing agent may be at low shear or high shear. Low shear mixing is preferred as it is less likely to cause degradation of oil components. The mixture of oil and inorganic complexing agent may be at a temperature just above the freezing point of the oil up to just below the decomposition temperature of the oil. In general mixing of the oil and inorganic complexing agent occurs at about 15°C to about 70°C, preferably from about 15°C to about 50°C and most preferably from about 15°C to about 30°C.

The mixing of the oil and inorganic complexing agent may be performed under an inert atmosphere such as nitrogen or carbon dioxide. The preparation of compositions containing oxidizable materials such as omega-3 acids is preferably conducted in an inert atmosphere.

In general, the process for increasing the viscosity of oils comprises, contacting the oil with an inorganic complexing agent, said complexing agent forming a viscosity increasing complex with the oil.

The oil compositions of increased viscosity may be stored for future use or formulated with conventional additives to prepare medicated and nonmedicated compositions which offer a variety of textures from a liquid to a grease, cream, lotion, ointment or other forms to suit particular applications. While the compositions of the present invention are ideally suited for nonaqueous applications, the structure formed between the oil and inorganic complexing agent is stable in the presence of water permitting complexed oil and water emulsions to be formed. The novel compositions of this invention may be utilized in pharmaceuticals, cosmetics, personal care products, foods, agriculture, lubricants and solvents.

In the following examples, the viscosities were measured on a Brookfield model LV-1 viscometer. These measurements represent relative and not "absolute" or "true" measures of viscosity. Viscosity measurements taken with the Brookfield using different spindles will vary. Example 2, Run 11 demonstrates that viscosities measured on spindle 2 will be, on the average, approximately 2.75 times higher than viscosities measured on spindle 3 for the same mixtures. The viscosities reported in the following examples must be interpreted in view of the above information.

The present invention is further illustrated by the following examples. All parts and percentages in the examples and throughout the specification and claims are by total weight of the composition unless otherwise indicated.

EXAMPLE 1

(Inventive Runs 1 to 8 and Controls)

This example demonstrates the effect of magnesium trisilicate (MgT) content on the viscosity of mineral oil (MO) and fish oil (FO) in centipoise (cps) as measured on a Brookfield model LV-1 viscometer.

The compositions of Table 1 where prepared by admixing magnesium trisilicate in the specified ratios with the particular oil until a uniform mixture was obtained:

3

## Table 1
### Viscosity of Magnesium Trisilicate
### Fish and Mineral Oil Suspensions

| Run | Ratio MgT/MO | Spindle # | Viscosity (cps) at Given RPM 6 | 12 | 30 | 60 |
|-----|------|-----------|------|------|------|------|
| 1 | 5/95 | 1 | 70 | 72.5 | 70 | 56 |
| 2 | 10/90 | 2 | 300 | 225 | 160 | 125 |
| 3 | 20/80 | 3 | 6,140 | 3,900 | 1,940 | 1,180 |
| 4 | 30/70 | 4 | -- | 22,000 | 16,400 | 9,300 |
| | Ratio MgT/FO | | | | | |
| 5 | 5/95 | 1 | 75 | 75 | 58 | 52 |
| 6 | 10/90 | 1 | 292 | 200 | 128 | 98 |
| 7 | 20/80 | 3 | 3,700 | 2,150 | 1,172 | 680 |
| 8 | 30/70 | 4 | 32,000 | 17,000 | 8,200 | 4,600 |

Controls

| | | | | | | |
|---|---|---|---|---|---|---|
| Fish Oil | | 1 | 35 | 30 | 28 | 30 |
| Mineral Oil | | 1 | 30 | 22.5 | 22 | 22 |

In both the fish oil and mineral oil, a 5:95 ratio of MgT to oil effectively doubles the viscosity of the uncomplexed oil. In Runs 1 to 8, as the concentration of MgT increases the viscosity of the mixtures increase dramatically. The suspensions formed in Runs 1 to 8 are shear thinning. Shear thinning results in a decrease in viscosity as the shear, in this case RPM of the spindle, is increased. This behavior is typical of suspensions which have structure. As the shear is increased, the structure is broken down thus giving a lower viscosity.
Runs 1 to 8 all demonstrate the presence of a gel-like structure and increased viscosity.

EXAMPLE 2

(Inventive Runs 9 to 14)

This example demonstrates the effect of inorganic complexing (IC) agents on the viscosity of mineral oil (MO) and fish oil (FO) as measured on a Brookfield model LV-1 viscometer.
The compositions of Table 2 were prepared by admixing the indicated inorganic complexing agent in the specified ratios with either fish oil or mineral oil until a uniform mixture was obtained.

4

Table 2

Viscosity of Fish and Mineral Oil Suspensions

| Run | Materials | Ratio IC/Oil | Spindle | Viscosity (cps) at Given RPM 6 | 12 | 30 | 60 |
|-----|-----------|--------------|---------|------|------|------|------|
| 9   | (1)/FO    | 20/80        | 3       | 400  | 400  | 200  | 180  |
| 10  | (1)/MO    | 20/80        | 2       | 1,000 | 700 | 430  | 295  |
| 11  | $CaCO_3$/MO | 20/80      | 2       | 3,700 | 2,175 | (2) | (2) |
|     |           |              | 3       | 1,300 | 825 | 440  | 420  |
| 12  | $Mg(OH)_2$/FO | 20/80    | 3       | 100  | 100  | 60   | 70   |
| 13  | $Mg(OH)_2$/MO | 20/80    | 2       | 150  | 150  | 120  | 90   |
| 14  | $CaCO_3$/FO | 20/80      | 2       | 150  | 125  | 100  | 80   |

Controls

| | | | | | | | |
|---|---|---|---|---|---|---|---|
| Fish Oil (FO) | | | 1 | 35 | 30 | 28 | 30 |
| Mineral Oil (MO) | | | 1 | 30 | 22.5 | 22 | 22 |

(1) = Aluminum hydroxide/calcium carbonate codried gel

(2) = Beyond range of instrument using spindle 2

Aluminum hydroxide/calcium carbonate codried gel shows a moderate effect on the viscosity of both mineral oil and fish oil. Calcium carbonate demonstrates a strong effect on the viscosity of mineral oil and a weak moderate effect on the viscosity of fish oil. Magnesium hydroxide demonstrates a weak moderate effect on the viscosity of both fish oil and mineral oil. All of the runs demonstrate shear thinning. Shear thinning is a drop in viscosity as shear, in this case RPM, is increased. The increases in viscosity of the inventive runs are caused by the structure developed around and between suspended particles. Calcium carbonate forms a strong structure containing composition with mineral oil but a weak structure containing composition with fish oil.

EXAMPLE 3

(Inventive Runs 15 to 20)

This example demonstrates the effect of the inorganic complexing agent magnesium trisilicate (MgT) on various oils.

The compositions of Table 3 were prepared by admixing magnesium trisilicate in the specified ratios with the particular oil until a uniform mixture was obtained.

## Table 3

### Mixtures of Magnesium Trisilicate and Various Oils

| Run | Oil | Ratio MgT/Oil | Product Consistency |
|-----|-----|---------------|---------------------|
| 15 | Peanut | 40:60 | Semisolid - salve |
| 16 | Safflower | 40:60 | Semisolid - salve |
| 17 | Corn | 40:60 | Semisolid - salve |
| 18 | Olive | 40:60 | Semisolid - salve |
| 19 | Castor | 20:80 | Viscous paste |
| 20 | Cod liver | 20:80 | Viscous paste |

All of the oils tested form a structure with magnesium trisilicate resulting in highly viscous compositions.

EXAMPLE 4

(Comparative Runs A to B)

This example demonstrates the effect of inorganic compounds which do not complex with oils.
The compositions of Table 4 were prepared by admixing various noncomplexing inorganic compounds (NIC) in the specified ratios with fish oil (FO) until a uniform mixture was obtained.

## Table 4

### Mixtures of Inorganic Compounds with Fish Oil

| Run | Inorganic Compound | Ratio NIC:FO | Product Consistency |
|-----|---------------------|--------------|---------------------|
| A | Veegum (1) | 20:80 | Particulates settle No significant viscosity increase |
| B | Colloidal silica | 20:80 | Particulates settle No significant viscosity increase |

(1) Veegum = complex magnesium aluminum silicate a standard article of commerce sold as Veegum by the R.T. Vanderbilt Co, Inc., New York, New York.

None of the inorganic compounds tested above formed complexes with fish oil. The compounds settled to the bottom of the solution and did not cause a significant increase in oil viscosity.

EXAMPLE 5

6

(Inventive Run 21)

This example demonstrates a method for preparing a palatable suspension of castor oil. The ingredients are mixed in the order indicated.

| No. | Ingredient | Percent (w/w) |
|-----|-----------|---------------|
| 1. | Castor Oil | 70.00 |
| 2. | Magnesium trisilicate | 19.47 |
| 3. | Maltodextrin | 5.00 |
| 4. | dl-alpha tocopherol | 0.03 |
| 5. | Flavor | 4.50 |
| 6. | Aspartame (sweetener) | 1.00 |

Procedure

The oil and magnesium trisilicate are mixed until a homogeneous mixture of increased viscosity is obtained. The maltodextrin, dl-alpha tocopherol, flavor and aspartame are added with continued mixing until a homogeneous suspension is formed.

The final suspension has a palatable mouth feel and a pleasant taste. The ingredients of the suspension will settle on standing, but rapidly resuspend with gentle mixing.

This invention being thus described, it will be obvious that the same may be varied in many ways. Such variations are not to be regarded as a departure from the spirit and scope of the invention and all such modifications are intended to be included within the scope of the following claims.

**Claims**

1. An oil composition having increased viscosity which comprises:
   an oil; and an inorganic complexing agent;
   said complexing agent being capable of forming a viscosity increasing complex with the oil.
2. The composition of claim 1 wherein the inorganic complexing agent is characterized by:
   being insoluble in oil; and
   forming a viscosity increasing complex with the oil.
3. The composition of claim 1 wherein the complexing agent is present in an amount sufficient to produce an increased viscosity but insufficient to produce a particulate solid.
4. The composition according to the claims 1 or 2 wherein the complexing agent is selected from the group consisting of magnesium trisilicate, calcium carbonate, calcium silicate, a codried gel of aluminum hydroxide and magnesium carbonate, magnesium carbonate, magnesium hydroxide, aluminum hydroxide, ground limestone, ground oyster shells and mixtures thereof.
5. The composition of claim 1 wherein the complexing agent is magnesium trisilicate.
6. The composition according to anyone of the claims 1 to 5 wherein the complexing agent is present in ratio to oil of about 1:99 to about 1.2:1 by weight.
7. The composition according to anyone of the claims 1 to 6 wherein the oil is selected from the group consisting of animal oil, vegetable oil, mineral oil, and mixtures thereof.
8. The composition according to anyone of the claims 1 to 6 wherein the oil is a marine oil.
9. The composition of claim 8 wherein the oil is a marine oil selected from the group consisting of fish oil, whale oil, seal oil, fish liver oil, oil containing at least one omega-3 fatty acid and mixtures thereof.
10. The composition of the claims 8 or 9 wherein the oil is a marine oil containing at least one omega-3 fatty acid.
11. The composition according to anyone of the claims 1 to 6 wherein the oil is a vegetable oil selected from the group consisting of castor, linseed, sunflower, soybean, olive, peanut, rapeseed, corn, safflower, cottonseed, coconut, palm, palm kernel, oils containing at least one omega-3 fatty acid and mixtures thereof.

12. The composition according to anyone of the claims 1 to 11 wherein the complexing agent has a particle size of about 0.1 micrometers to about 300 micrometers.

13. A process for producing an oil composition having increased viscosity as claimed in anyone of the claims 1 to 12, which comprises: contacting the oil with an inorganic complexing agent, said complexing agent forming a viscosity increasing complex with the oil.

14. The process of claim 13 wherein the complexing agent is characterized by:
    being insoluble in oil; and
    forming an increased viscosity complex with the oil.

15. An oil composition having increased viscosity which comprises:
    an oil; and an inorganic complexing agent ;
    said complexing agent being capable of forming a viscosity increasing complex with the oil wherein said oil is fish oil.

16. An oil composition having increased viscosity which comprises:
    an oil; and an inorganic complexing agent;
    said complexing agent being capable of forming a viscosity increasing complex with the oil wherein said oil is castor oil.

17. An oil composition having increased viscosity which comprises:
    an oil; and an inorganic complexing agent;
    said complexing agent being capable of forming a viscosity increasing complex with the oil wherein said oil is cod liver oil.